(19) Europäisches Patentamt
European Patent Office
Office européen des brevets

(11) Publication number : **0 400 889 B1**

(12) # EUROPEAN PATENT SPECIFICATION

(45) Date of publication of patent specification : **19.01.94 Bulletin 94/03**

(51) Int. Cl.5 : **C07C 279/14,** C07C 277/08

(21) Application number : **90305617.4**

(22) Date of filing : **23.05.90**

(54) **Crystalline deoxyspergualin and process for its preparation.**

(30) Priority : **29.05.89 JP 132878/89**
**29.05.89 JP 132879/89**

(43) Date of publication of application : **05.12.90 Bulletin 90/49**

(45) Publication of the grant of the patent : **19.01.94 Bulletin 94/03**

(84) Designated Contracting States : **DE ES FR GB IT NL SE**

(56) References cited :
**EP-A- 0 094 632**
**FR-A- 2 514 350**
**CHEMICAL ABSTRACTS,vol. 109, no. 5, 1 August 1988, page 657, abstract no. 38200x, Columbus, Ohio, US; Y. UMEDA et al.: "Synthesis and antitumor activity of spergualin analogs. III. Novel method for synthesis of optically active 15-deoxyspergualin and 15-deoxy-11-0-methylspergualin"**

(73) Proprietor : **TAKARA SHUZO CO. LTD.**
**609 Takenakacho**
**Fushimi-ku Kyoto (JP)**
Proprietor : **NIPPON KAYAKU KABUSHIKI KAISHA**
**11-2, Fujimi 1-chome Chiyoda-ku**
**Tokyo 102 (JP)**

(72) Inventor : **Ikai, Katsushige**
**9-421-45 Kibogaoka Honmachi, Konan-cho**
**Koga-gun, Shiga-ken (JP)**
Inventor : **Umeda, Yoshihisa**
**2-27-21 Jinryo**
**Otsu-shi, Shiga-ken (JP)**
Inventor : **Saino, Tetsushi**
**5-11-14-101, Hachioji**
**Yono-shi, Saitama-ken (JP)**
Inventor : **Moriguchi, Makoto**
**52-5 Kitazutsumi, Naka**
**Jyoyo-shi, Kyoto-fu (JP)**
Inventor : **Kato, Ikunoshin**
**1-1-150 Nanryo-cho**
**Uji-shi, Kyoto-fu (JP)**

(74) Representative : **Marlow, Nicholas Simon et al**
**Reddie & Grose 16, Theobalds Road**
**London WC1X 8PL (GB)**

EP 0 400 889 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to crystals of N-[4-(3-aminopropyl)aminobutyl]-2-(7-guanidinoheptanamide)-2-hydroxy-ethanamide trihydrochloride (hereinafter referred to as deoxyspergualin hydrochloride), which has antitumor activity and immunosuppressive activity, and a process for the preparation of the same.

Deoxyspergualin with the formula

$$H_2N\underset{\underset{NH}{\|}}{C}NH(CH_2)_6CONH\underset{\underset{OH}{|}}{C}HCONH(CH_2)_4NH(CH_2)_3NH_2$$

has excellent antitumor activity against various animal tumors and excellent immunosuppressive activity, and therefore it is a useful compound for clinical use. Deoxyspergualin has conventionally been prepared as the trihydrochloride salt.

For the preparation of deoxyspergualin hydrochloride, a synthetic method using the acid-catalyzed dehydration condensation or 7-guanidinoheptanamide with glyoxylylspermidine is known [U.S. Patent Nos. 4,518,532 and 4,603,015].

The powder of deoxyspergualin hydrochloride obtained by the above-mentioned synthetic method has undesirable properties such as hygroscopicity and heat instability. This invention is to provide crystals of deoxyspergualin hydrochloride to improve the hygroscopic and heat-unstable properties of deoxyspergualin hydrochloride.

The present invention provides crystals of deoxyspergualin trihydrochloride. The present invention also provides processes for the preparation of crystals of deoxyspergualin trihydrochloride characterized by crystallizing a powder or water-suspension syrup of deoxyspergualin trihydrochloride in an atmosphere of relative humidity of 52% or less or in the presence of water and ethanol.

The present invention further provides a pharmaceutical composition comprising crystalline deoxyspergualin trihydrochloride.

We have found for the first time that deoxyspergualin hydrochloride can be crystallized, and that the crystals have remarkably improved hygroscopicity, handling properties and heat stability as compared with the powder or lyophilized powder which has been conventionally prepared and used. This invention is based on such finding.

In this invention, the term "powder of deoxyspergualin hydrochloride" means the powder obtained from a water suspension syrup of deoxyspergualin hydrochloride by drying under reduced pressure, or the lyophilized powder. The term "water suspension syrup of deoxyspergualin hydrochloride" means a syrupy residue obtained by concentrating an aqueous solution of deoxyspergualin hydrochloride under reduced pressure. Usually the content of water in such syrup is 30% or less.

The crystalline deoxyspergualin hydrochloride of this invention has improved hygroscopicity and heat stability, and the crystals are in α-form or β-form.

α-form crystals of deoxyspergualin hydrochloride can be prepared in the following manner:

Deoxyspergualin hydrochloride itself can be prepared by various methods, and one of the methods is described in Example 4 of U.S. Patent No. 4,518,532. The present invention is not limited to any particular method for the preparation of deoxyspergualin hydrochloride.

When the powder of deoxyspergualin hydrochloride is left standing in a low humidity atmosphere, at a relative humidity (hereafter referred to as RH) of 52% or below, and at a suitable temperature, the powder is crystallized to the α-form crystals of deoxyspergualin hydrochloride of this invention.

The temperature used for the crystallization is not limited to any particular temperature or range of temperatures, provided that the temperature does not cause the degradation of deoxyspergualin hydrochloride, and temperature for practical use is in the range of 0°C to 30°C. The time required for the crystallization is in the range of 2 to 20 days, and is usually about 10 days, depending upon the amount of the powder and humidity.

The α-form crystals can also be obtained from a syrup of deoxyspergualin hydrochloride. Thus, an aqueous solution of deoxyspergualin hydrochloride is concentrated to yield a syrup, which is then left standing in a low humidity atmosphere, at a RH of 52% or below to give α-form crystals. The temperature and the time for crystallization may be similar to those mentioned above, but the time may be shortened by stirring the syrup during the crystallization or by adding a suitable amount of α-form crystals into the syrup.

The most practical process for the preparation of α-form crystals is as follows. To a water suspension syrup with a water content of 20% (w/w) or below is added a small amount of α-form crystals prepared in advance, and the syrup is stirred at a RH of 30% or less until it solidifies in a few hours. The crystallization becomes

complete when stored in a silica gel desiccator.

The physicochemical properties of α-form crystals of deoxyspergualin hydrochloride are as follows.

1. X-ray diffraction pattern:

An X-ray diffraction apparatus (Shimadzu XD-610) equipped with a Cu X-ray tube, graphite monochrometer and scintillation counter for detection was used to study the X-ray diffraction pattern, which is shown in Figure 1 and Table 1.

In Figure 1 is shown the X-ray powder diffraction pattern of α-form crystals of deoxyspergualin hydrochloride with the diffraction angle (degrees) on the abscissa, and the intensity (Kcps) on the ordinate.

Table 1. X-ray diffraction pattern of α-form crystals

| d (Å) | $I/I_1$ | d (Å) | $I/I_1$ |
|---|---|---|---|
| 11.78 | 0.46 | 4.04 | 0.76 |
| 10.64 | 0.53 | 3.90 | 0.91 |
| 7.49 | 0.34 | 3.78 | 0.76 |
| 5.03 | 0.36 | 3.50 | 1.00 |
| 4.72 | 0.51 | 3.39 | 0.95 |
| 4.39 | 0.84 | 3.12 | 0.64 |

X-ray source: Cu X-ray tube. 40 KV, 30 mA. $\lambda$=1.54051

Filter: Graphite monochrometer

2. Thermoanalysis:

Results of differential thermal analysis (DTA) and thermogravimetry (TG), which were obtained simultaneously by using Rigaku Denki (Model TAS-100), are shown in Figure 2 for α-form crystals of deoxyspergualin hydrochloride. Results are depicted with temperature (°C) on the abscissa and weight (mg) or electron volts (μV) on the ordinate.

3. Hygroscopicity:

To evaluate the hygroscopicity of the powder and α-form crystals of deoxyspergualin hydrochloride, the change of the weight of each sample was measured after storage in different RH (%) for 24 hrs. The results are shown in Table 2.

Table 2. Changes in weight under conditions of different humidity

| RH (%) | Increase in weight (%) | |
|---|---|---|
| | Powder | α-form crystals |
| 14 | 1.5 | - |
| 31 | 5.9 | -0.8 |
| 43 | 9.1 | -0.4 |
| 52 | 12.6 | -0.2 |
| 64 | 17.9 | 17.8 |

As shown in Table 2, the α-form crystals of deoxyspergualin hydrochloride did not absorb water at RH of 52% or below, showing improvement in hygroscopity as compared with powder.

4. Heat stability:

To evaluate the heat stability or α-form crystals of deoxyspergualin hydrochloride in comparison with that of the powder, samples were stored at 50°C for 6 days and were analyzed by high-pressure liquid chromatography (HPLC), and the amount of undecomposed substance was measured. The results are shown in Table 3.

Table 3. Comparison of heat stability

| | Powder | α-form crystals |
|---|---|---|
| Amount (%) | 83 | 100.0 |

Note: $\text{Amount (\%)} = \frac{\text{Amount after storage}}{\text{Initial amount}} \times 100$

As shown in Table 3, the α-form crystals show remarkable improvement in heat stability.

β-form crystals of deoxyspergualin hydrochloride can be prepared in the following manner. Deoxyspergualin hydrochloride itself can be prepared by various methods, and one of the methods is described in Example 4 of U.S Patent No. 4,518,532. The present invention is not limited to any particular method for the preparation of deoxyspergualin hydrochloride. When the powder of deoxyspergualin hydrochloride thus obtained is left standing in a mixture of water and ethanol there are obtained β-form crystals of deoxyspergualin hydrochloride. The concentration of ethanol in the solution to be used for the crystallization is usually in the range of 85 to 99.5% (v/v), and preferably in the range of 90 to 98% (v/v).

The temperature for the crystallization is not critical, provided that the temperature does not cause the degradation of deoxyspergualin hydrochloride, and usually it is in the range of -20 to 50°C, and preferably from 5°C to the room temperature. The time needed for the crystallization is in the range of 2 to 20 days, depending upon the amount of the hydrochloride used and usually, it is about 10 days. At the early stage of the crystallization, seeding a small portion of β-form crystals obtained in advance may promote crystallization.

In the above method, a syrup (water suspension syrup) of deoxyspergualin hydrochloride may be used in place of powder of deoxyspergualin hydrochloride. In this case the amount of water contained in the syrup should be taken into consideration so that the concentration of ethanol in the crystallization system is 85-99.5%, preferably 90-98% (v/v).

The proportion of ethanol used for crystallization to deoxyspergualin hydrochloride is not restricted, but is

usually in the range of ratios of 3:1 to 20:1 (v/w).

Stirring during the crystallization reduces the time for crystallization; this time comes to be in the range of a few hours to several days. β-form crystalls thus formed can be collected by any ordinary method such as filtration under reduced pressure or filtration which makes use of centrifugal force.

The physicochemical properties of β-form crystals of deoxyspergualin hydrochloride are as follows.

1. X-ray diffraction pattern:

An X-ray diffraction apparatus (Shimadzu XD-3A) equipped with a Cu X-ray tube, graphite monochrometer and scintillation counter for detection was used to study the X-ray diffraction pattern, which is shown in Figure 3 and Table 4.

In Figure 3 is shown the X-ray powder diffraction pattern of β-form crystals of deoxyspergualin hydrochloride with the diffraction angle (degrees) on the abscissa, and the intensity (Kcps) on the ordinate.

Table 4. X-ray diffraction pattern of β-form crystals

| d (Å) | $I/I_1$ | d (Å) | $I/I_1$ |
|---|---|---|---|
| 15.77 | 0.32 | 4.14 | 0.70 |
| 10.91 | 0.42 | 3.66 | 1.00 |
| 4.88 | 0.39 | 3.31 | 0.52 |
| 4.67 | 0.53 | 3.21 | 0.52 |

X-ray source: Cu X-ray tube. 40 KV, 30 mA. $\lambda$ = 1.54051

Filter: Graphite monochrometer

2. Thermoanalysis:

Results of differential thermal analysis (DTA) and thermogravimetry (TG), which were obtained simultaneously by using Shimadzu apparatus (model DTG-40) are shown in Figure 4 for β-form crystals of deoxyspergualin hydrochloride. Results are depicted with temperature (°C) on the abscissa and weight (mg) or electron volts (μV) on the ordinate.

3. Hygroscopicity:

To evaluate the hygroscopicity of the powder and β-form crystals of deoxyspergualin hydrochloride, the change in the weight of each sample was measured after storage in different RH (%) for 24 hrs. The results are shown in Table 5.

Table 5. Changes in weight under conditions of different humidity

| RH (%) | Increase in weight (%) | |
|---|---|---|
| | Powder | β-form crystals |
| 31 | 5.9 | 1.7 |
| 43 | 9.1 | 2.6 |
| 52 | 12.6 | 3.8 |
| 64 | 17.9 | 7.3 |
| 76 | 28.3 | 14.0 |

As shown in Table 5, the change in the weight of the β-form crystals was less at all humidities than that of the powder. Furthermore, β-form crystals did not deliquesce at RH of 64%, but the powder deliquesced at this humidity.

4. Heat stability:

To evaluate the heat stability of β-form crystals of deoxyspergualin hydrochloride in comparison with that of the powder, samples were stored at 50°C for 6 days, and analyzed by HPLC to measure the amount of undecomposed substance. The results are shown in Table 6.

Table 6. Comparison of heat stability

| | Powder | β-form crystals |
|---|---|---|
| Amount (%) | 89.8 | 100.6 |

Note: $\text{Amount (\%)} = \frac{\text{Amount after storage}}{\text{Initial amount}} \times 100$

As apparent from Table 6, the β-form crystals shows remarkable improvement in heat stability.

The invention will be further explained by means of the following Examples. It should be understood however that the invention is not limited to these examples.

Example 1

Powder of deoxyspergualin hydrochloride (500 mg) was left standing at 25°C and RH of 52% for 11 days to allow crystallization. The crystals (506 mg) obtained showed the X-ray diffraction pattern typical of α-form crystals depicted in Figure 1.

Example 2

A lyophilized powder of deoxyspergualin hydrochloride (500 mg) was left standing at 25°C and RH of 43% for 11 days to yield α-form crystals (510 mg).

Example 3

A lyophilized powder of deoxyspergualin hydrochloride (500 mg) was left standing at 25°C and RH of 31%

for 11 days to yield α-form crystals (509 mg).

Example 4

An aqueous solution (100 ml) of 40 g of deoxyspergualin hydrochloride was concentrated under reduced pressure to yield a syrupy residue (46 g). To the residue was added 20 mg of α-form crystals, and the mixture was stirred at a RH of 15% at the room temperature. After being stirred for 2 hr, the solidified residue was left standing in a silica gel desiccator for 3 days to yield α-form crystals (42 g).

Example 5

To a vial containing 100 mg of powder of deoxyspergualin hydrochloride was added 0.5 ml of 95% (v/v) aqueous ethanol, and the solution was left standing at 5°C for 3 days. The crystals formed in the solvent were collected by filtration under reduced pressure, and dried under reduced pressure to yield 55 mg of β-form crystals. X-ray diffraction pattern of the crystals obtained showed the X-ray diffraction pattern typical of β-form crystals depicted in Figure 3.

Example 6

An aqueous solution (100 ml) containing 20 g of deoxyspergualin hydrochloride was concentrated under reduced pressure to yield 24 g of a syrupy residue. To the residue was added 75 ml of ethanol, and 20 mg of β-form crystals was seeded. The mixture was left standing at 5 °C for 9 days. The crystals were collected as in Example 5 and dried, giving β-form crystals (16 g).

Example 7

An aqueous solution (500 ml) of deoxyspergualin hydrochloride (150 g) was concentrated under reduced pressure to obtain a syrupy residue (175 g). To the residue was added 1 liter of ethanol, and the solution was stirred at 5°C for 2 days. The crystals formed were collected by centrifugal filtration, and dried under reduced pressure, giving 129 g of β-form crystals.

Example 8

An aqueous solution (4 liters) of deoxyspergualin hydrochloride (950 g) was concentrated under reduced pressure to obtain a syrupy residue (1120 g). To the residue was added 1.5 liter of ethanol, and the suspension was transferred to a vessel (10 liters volume) for crystallization. To the mixture were added 6.5 liters of ethanol and 2 g of β-form crystals with stirring, and stirring was continued at 15°C for 4 days. The crystals were collected by centrifugal filtration, and dried under reduced pressure, yielding 880 g of β-form crystals.

The crystalline deoxyspergualin hydrochloride of the present invention is novel and possesses improved hygroscopicity and heat stability, which results in easier handling of deoxyspergualin during formulation and storage.

## Claims

1. Crystalline deoxyspergualin trihydrochloride.

2. Crystalline deoxyspergualin trihydrochloride according to claim 1 in the form of a-form crystals having an X-ray diffraction pattern as follows:

| d (Å) | I/I₁ | d (Å) | I/I₁ |
|---|---|---|---|
| 11.78 | 0.46 | 4.04 | 0.76 |
| 10.64 | 0.53 | 3.90 | 0.91 |
| 7.49 | 0.34 | 3.78 | 0.76 |
| 5.03 | 0.36 | 3.50 | 1.00 |
| 4.72 | 0.51 | 3.39 | 0.95 |
| 4.39 | 0.84 | 3.12 | 0.64 |

3. Crystalline deoxyspergualin trihydrochloride according to claim 1 in the form of β-form crystals having an X-ray powder diffraction pattern as follows:

| d (Å) | $I/I_1$ | d (Å) | $I/I_1$ |
|---|---|---|---|
| 15.77 | 0.32 | 4.14 | 0.70 |
| 10.91 | 0.42 | 3.66 | 1.00 |
| 4.88 | 0.39 | 3.31 | 0.52 |
| 4.67 | 0.53 | 3.21 | 0.52 |

4. A process for the preparation of a-form crystals of deoxyspergualin trihydrochloride, which comprises crystallizing a powder or water-suspension syrup of deoxyspergualin trihydrochloride in an atmosphere having a relative humidity of 52% or less.

5. A process according to claim 4 in which crystallization is carried out at a temperature of from 0°C to 30°C.

6. A process for the preparation of β-form crystals of deoxyspergualin trihydrochloride, which comprises crystallizing a powder or water-suspension syrup of deoxyspergualin trihydrochloride in the presence of water and ethanol.

7. A process according to claim 6 in which crystallization is carried out at a temperature of from -20 to 50°C.

8. A process according to any of claims 4 to 7 in which crystallization takes place over from 2 to 20 days, preferably over 10 days.

9. A process according to any of claims 4 to 8 in which the water content of the water suspension syrup is 30% or less.

10. A pharmaceutical composition comprising crystalline deoxyspergualin trihydrochloride according to any of claims 1 to 4.

**Patentansprüche**

1. Kristallines Desoxyspergualin-Trihydrochlorid.

2. Kristallines Desoxyspergualin-Trihydrochlorid nach Anspruch 1 in Form von α-Kristallen folgenden Röntgenbeugungsdiagramms:

| d (Å) | $I/I_1$ | d (Å) | $I/I_1$ |
|---|---|---|---|
| 11.78 | 0.46 | 4.04 | 0.76 |
| 10.64 | 0.53 | 3.90 | 0.91 |
| 7.49 | 0.34 | 3.78 | 0.76 |
| 5.03 | 0.36 | 3.50 | 1.00 |
| 4.72 | 0.51 | 3.39 | 0.95 |
| 4.39 | 0.84 | 3.12 | 0.64 |

3. Kristallines Desoxyspergualin-Trihydrochlorid nach Anspruch 1 in Form von β-Kristallen folgenden Röntgenbeugungsdiagramms:

| d (Å) | $I/I_1$ | d (Å) | $I/I_1$ |
|---|---|---|---|
| 15.77 | 0.32 | 4.14 | 0.70 |
| 10.91 | 0.42 | 3.66 | 1.00 |
| 4.88 | 0.39 | 3.31 | 0.52 |
| 4.67 | 0.53 | 3.21 | 0.52 |

4. Verfahren zur Herstellung von α-Form-Kristallen von Desoxyspergualin-Trihydrochlorid, bei dem ein Pulver oder ein Wasser-Suspensionssirup von Desoxyspergualin-Trihydrochlorid in einer Atmosphäre mit einer relativen Feuchtigkeit von 52 % oder weniger kristallisiert wird.

5. Verfahren nach Anspruch 4, worin die Kristallisation bei einer Temperatur von 0°C bis 30°C durchgeführt wird.

6. Verfahren zur Herstellung von β-Form-Kristallen von Desoxyspergualin-Trihydrochlorid, bei dem ein Pulver oder ein Wasser-Suspensionssirup von Desoxyspergualin-Trihydrochlorid in Gegenwart von Wasser vund Ethanol kristallisiert wird.

7. Verfahren nach Anspruch 6, worin die Kristallisation bei einer Temperatur von -20°C bis 50°C durchgeführt wird.

8. Verfahren nach einem der Ansprüche 4 bis 7, worin die Kristallisation über 2 bis 20 Tage, vorzugsweise über 10 Tage, stattfindet.

9. Verfahren nach einem der Ansprüche 4 bis 8, worin der Wassergehalt des Wasser-Suspensionssirups 30 % oder weniger beträgt.

10. Pharmazeutische Zusammensetzung, umfassend kristallines Desoxyspergualin-Trihydrochlorid nach einem der Ansprüche 1 bis 4.

## Revendications

1. Trichlorhydrate de désoxyspergualine cristallin.

2. Trichlorhydrate de désoxyspergualine cristallin suivant la revendication 1, sous forme de cristaux de type α ayant le diagramme de diffraction des rayons X suivant :

| d (Å) | $I/I_1$ | d (Å) | $I/I_1$ |
|---|---|---|---|
| 11,78 | 0,46 | 4,04 | 0,76 |
| 10,64 | 0,53 | 3,90 | 0,91 |
| 7,49 | 0,34 | 3,78 | 0,76 |
| 5,03 | 0,36 | 3,50 | 1,00 |
| 4,72 | 0,51 | 3,39 | 0,95 |
| 4,39 | 0,84 | 3,12 | 0,64 |

3. Trichlorhydrate de désoxyspergualine cristallin suivant la revendication 1, sous forme de cristaux de type β ayant le diagramme de diffraction des rayons X sur poudre suivant :

| d (Å) | $I/I_1$ | d (Å) | $I/I_1$ |
|---|---|---|---|
| 15,77 | 0,32 | 4,14 | 0,70 |
| 10,91 | 0,42 | 3,66 | 1,00 |
| 4,88 | 0,39 | 3,31 | 0,52 |
| 4,67 | 0,53 | 3,21 | 0,52 |

4. Procédé de préparation de cristaux de forme α de trichlorhydrate de désoxyspergualine, qui comprend la cristallisation d'une poudre ou d'un sirop consistant en une suspension aqueuse de trichlorhydrate de désoxyspergualine dans une atmosphère ayant une humidité relative égale ou inférieure à 52 %.

5. Procédé suivant la revendication 4, dans lequel la cristallisation est effectuée à une température de 0°C à 30°C.

6. Procédé de préparation de cristaux de forme β de trichlorhydrate de désoxyspergualine, qui comprend la cristallisation d'une poudre ou d'un sirop consistant en une suspension aqueuse de trichlorhydrate de désoxyspergualine en présence d'eau et d'éthanol.

7. Procédé suivant la revendication 6, dans lequel la cristallisation est effectuée à une température d'au moins 20 à 50°C.

8. Procédé suivant l'une quelconque des revendications 4 à 7, dans lequel la cristallisation s'effectue en un temps de 2 à 20 jours, de préférence de 10 jours.

9. Procédé suivant l'une quelconque des revendications 4 à 8, dans lequel la teneur en eau du sirop consistant en une suspension aqueuse est égale ou inférieure à 30 %.

10. Composition pharmaceutique comprenant du trichlorhydrate de désoxyspergualine cristallin suivant l'une quelconque des revendications 1 à 4.

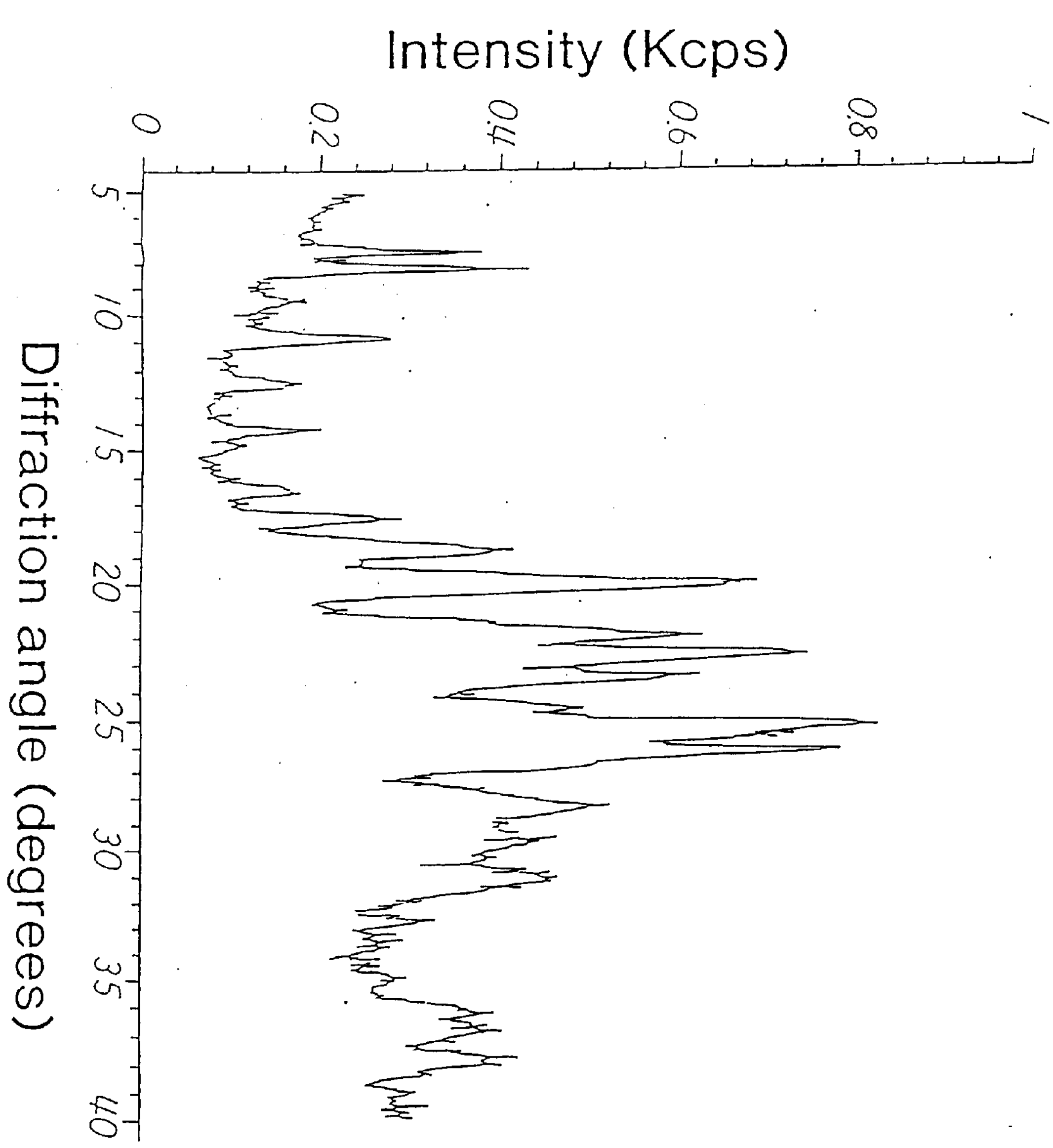
Intensity (Kcps)
0
0.2
0.4
0.6
0.8
1
5
10
15
20
25
30
35
40
Diffraction angle (degrees)

Figure 1.

Figure 2.

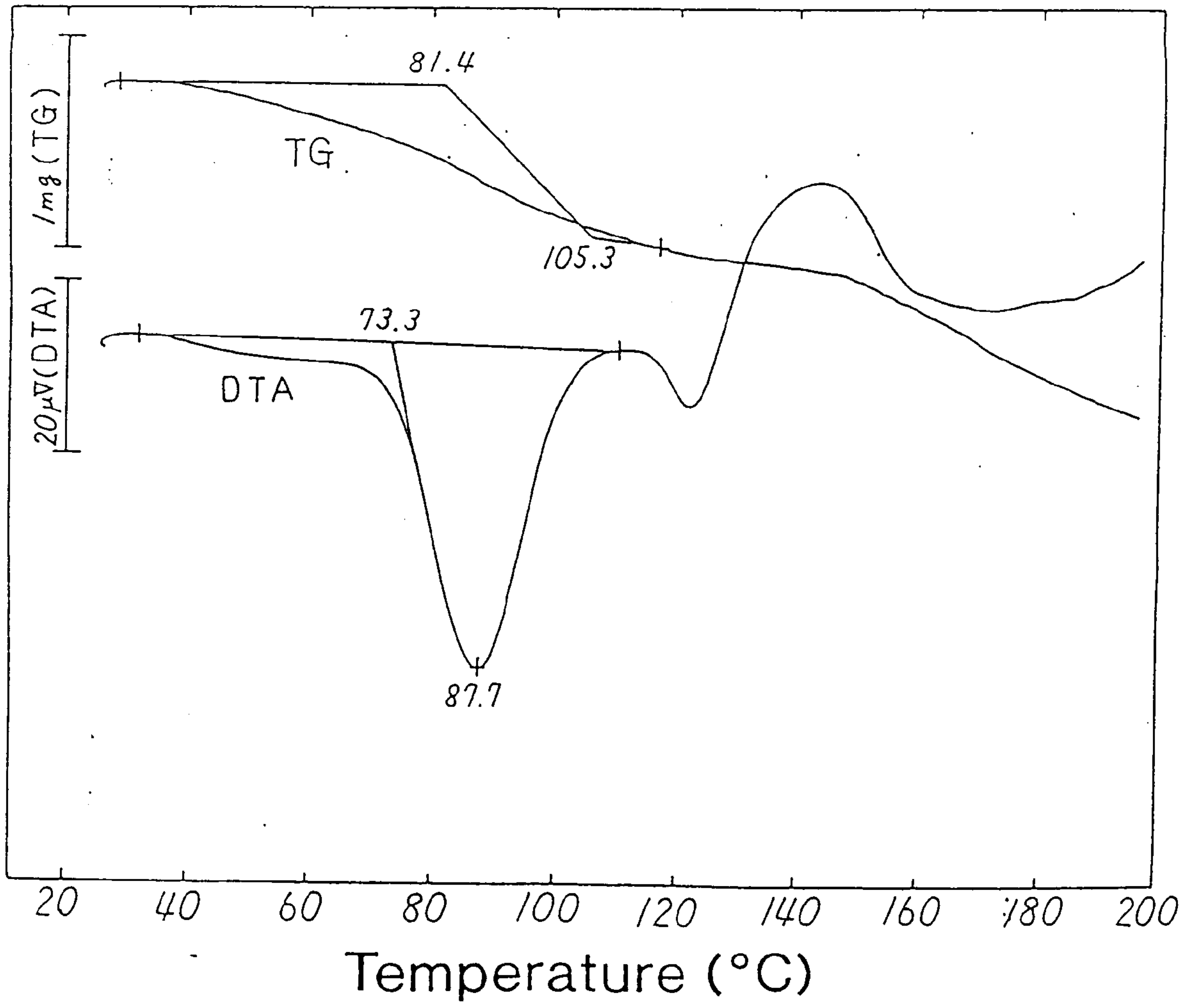
81.4
TG
1mg (TG)
105.3
73.3
DTA
20μV(DTA)
87.7
20
40
60
80
100
120
140
160
180
200
Temperature (°C)

Figure 3.

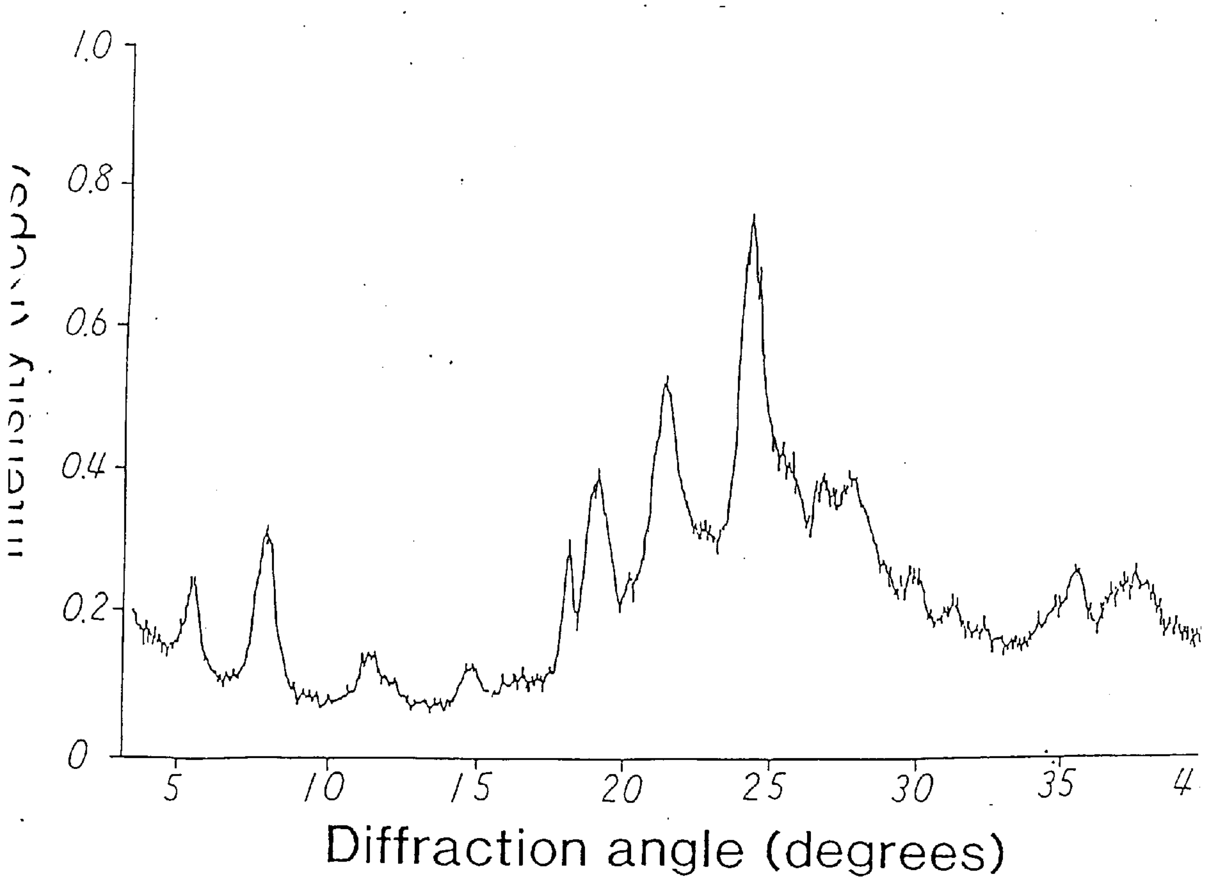

Figure 4.

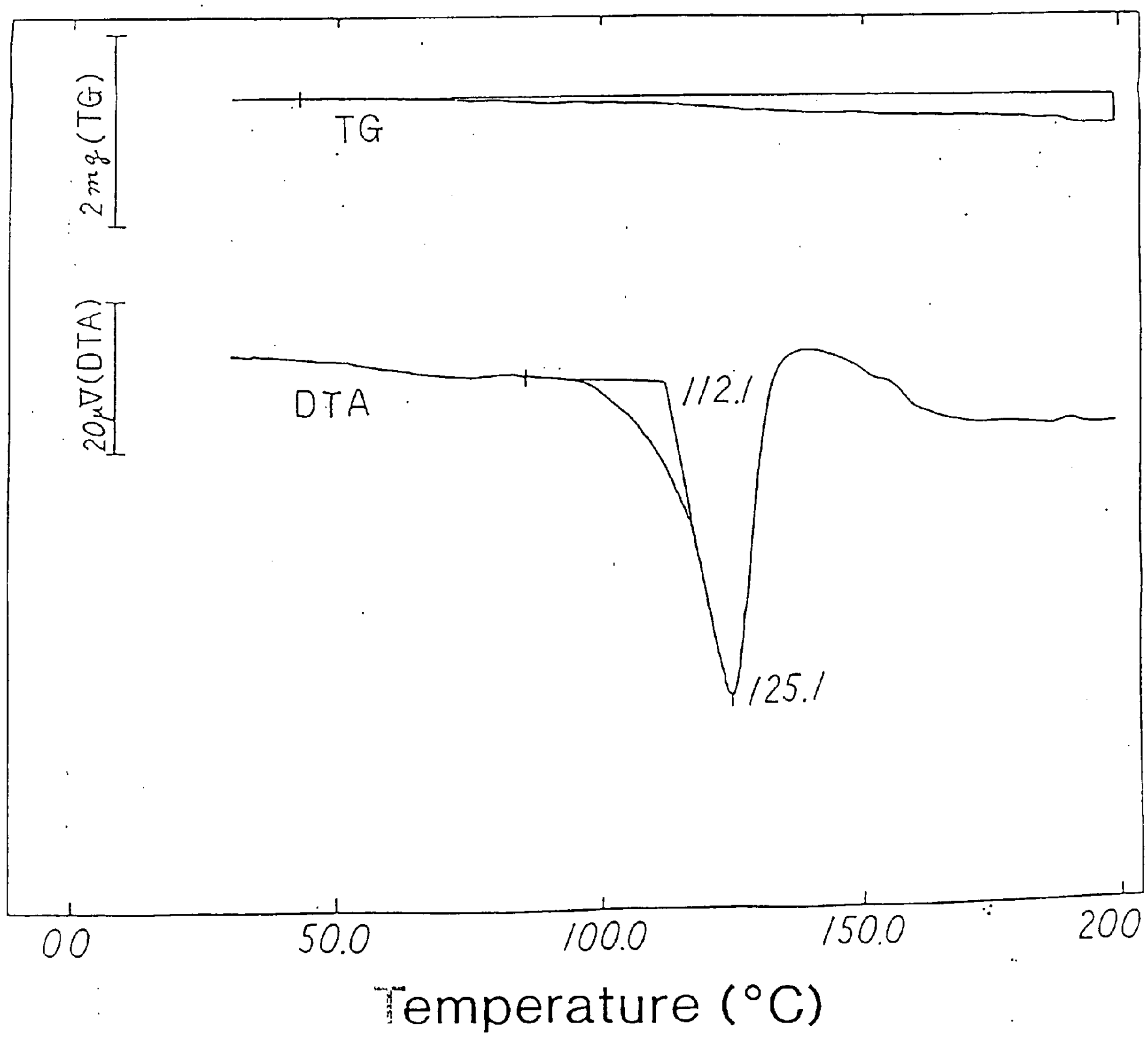
2mg (TG)
TG
20μV (DTA)
DTA
112.1
125.1
0.0
50.0
100.0
150.0
200
Temperature (°C)